# EUROPEAN PATENT APPLICATION

(11) **EP 1 894 599 A1**
(43) Date of publication of application: **05.03.2008**
(21) Application number: 07017121.0
(22) Date of filing: 31.08.2007
(51) Int. Cl.: A61N 1/05, A61N 1/36

(54) **Vision regeneration assisting apparatus**

(30) Priority: 31.08.2006 JP 2006237007
(71) Applicant: Nidek Co., Ltd., Gamagori-shi Aichi-ken 443-0038 (JP)
(72) Inventor: Terasawa, Yasuo, Obu-shi, Aichi-ken 474-0027 (JP); Shodo, Kenzo, Kyoto-shi, Kyoto-fu 615-8223 (JP); Ohta, Jun, Soraku-gun, Kyoto-fu 619-0232 (JP)
(74) Representative: Hofer, Dorothea

(57) **Abstract**

A vision regeneration assisting apparatus for regenerating vision by applying electrical stimulation to cells that compose a retina, the apparatus comprising: a substrate formed in a thin plate shape of insulating and bendable material, provided with a plurality of first lead wires being wired therein and an electrode for outputting an electrical stimulation pulse signal being provided at one end of each of the first lead wires; a multiplexer for distributing the electrical stimulation pulse signal to each electrode; a base formed of sealing and insulating material, one face of which being united to the substrate, and the other face of which being united with the multiplexer, and provided with a plurality of second lead wires for connecting the multiplexer and each of the first lead wires in the substrate being wired therein; and a cap formed of sealing material, and united to the base so that the multiplexer is to be hermetically sealed. In this manner, a circuit of an internal device installed at the patient's intra-body (intra-eye) can be easily and reliably hermetically sealing.

## Description

### BACKGROUND OF THE INVENTION

### 1. Field of the Invention

The present invention relates to a vision regeneration (revival) assisting apparatus for regenerating (reviving) patient's vision.

### 2. Description of the Related Art

In recent years, a vision regeneration (revival) assisting apparatus has been proposed for regenerating (reviving) patient's lost vision by applying electrical stimulation (electrical stimulation pulse signal) to cells that compose a retina from an electrode installed at an intra-body (intra-eye) of a patient who lost eyesight. Such apparatus has an internal device in which a number of electrodes and a circuit are disposed on a substrate, and this internal device is installed at the patient's intra-body (intra-eye) .

Portions other than the electrodes of the internal device (in particular, the circuit) are sealed by coating of biocompatible resin so as to be protected from invasion of body fluid. However, resin sealing is easy in processing, whereas there is possibility not to ensure a sealing property over a long period of time. Japanese Patent Application Laid-openNo. 2006-187409 discloses that hermetic sealing is carried out by coating (plating) of biocompatible metal, and then, sealing is carried out by resin coating. However, metal sealing can ensure a sealingpropertyover a longperiodof time, whereas processing with thickness for obtaining a sufficient sealing property is difficult.

Inaddition, Japanese Patent Application Laid-open No. 9-173477 discloses that a circuit of an intra-body implant type treatment device such as a heart pacemaker are placed in a metallic container, and then, hermetically sealed, although it is not a vision regeneration assisting apparatus.

### SUMMARY OF THE INVENTION

It is a technical problem of the present invention to provide a vision regeneration assisting apparatus which is capable of easily and reliably hermetically sealing the circuit of the internal device installed at the patient's intra-body (intra-eye).

In order to solve the problem described above, the present invention is characterized by having the following features.
(1) A vision regeneration assisting apparatus for regenerating vision by applying electrical stimulation to cells that compose a retina, the apparatus comprising:
   a substrate formed in a thin plate shape of insulating and bendable material, provided with a plurality of first lead wires being wired therein and an electrode for outputting an electrical stimulation pulse signal being provided at one end of each of the first lead wires;
   a multiplexer for distributing the electrical stimulation pulse signal to each electrode;
   a base formed of sealing and insulating material, one face of which being united to the substrate, and the other face of which being united with the multiplexer, and provided with a plurality of second lead wires for connecting the multiplexer and each of the first lead wires in the substrate being wired therein; and
   a cap formed of sealing material, and united to the base so that the multiplexer is to be hermetically sealed.
(2) The vision regeneration assisting apparatus according to (1), wherein the base is formed of sealing and insulating ceramics, and the cap is formed of sealing metal or sealing ceramics.
(3) The vision regeneration assisting apparatus according to (1), wherein, on the base, the multiplexer is united by flip chip attaching.
(4) The vision regeneration assisting apparatus according to (1), wherein the substrate is formed in a shape of an elongated thin plate, the electrode is provided at a front side of the substrate, and the base is united to a rear side of the substrate.
(5) The vision regeneration assisting apparatus according to (4), wherein the electrode is provided on one face of the substrate, and the base is united to the other face of the substrate.
(6) The vision regeneration assisting apparatus according to (5), the front side of the substrate is installed by inserting into an eye, and the rear side of the substrate is installed outside of the eye.
(7) The vision regeneration assisting apparatus according to (1),
wherein the apparatus is an extra-body imaging type apparatus, and further, has a photographing unit for photographing an object to be visually recognized by a patient outside of a patient's body.

### BRIEF DESCRIPTION OF THE DRAWINGS

FIG. 1 is a schematic view of an external device of a vision regeneration assisting apparatus according to an embodiment of the present invention.

FIG. 2A and FIG. 2B are schematic views of an internal device of the vision regeneration assisting apparatus.

FIG. 3 is a schematic cross section of the vicinity of a multiplexer of the internal device.

FIG. 4A, FIG. 4B, and FIG. 4C are illustrative views of hermetic sealing of the multiplexer.

FIG. 5 is a view showing a state in which the internal device is installed at an intra-body (intra-eye) of a patient.

FIG. 6 is a schematic block diagram of a control system of the vision regeneration assisting apparatus.

### DESCRIPTION OF THE PREFERRED EMBODIMENTS

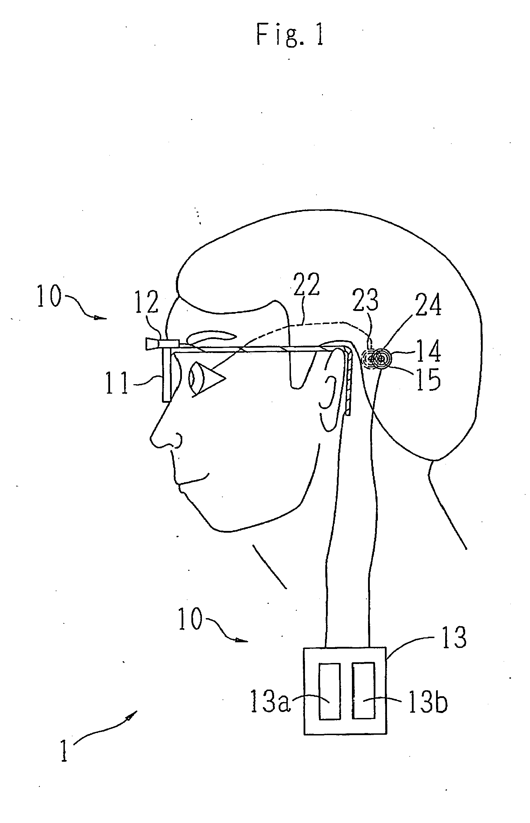

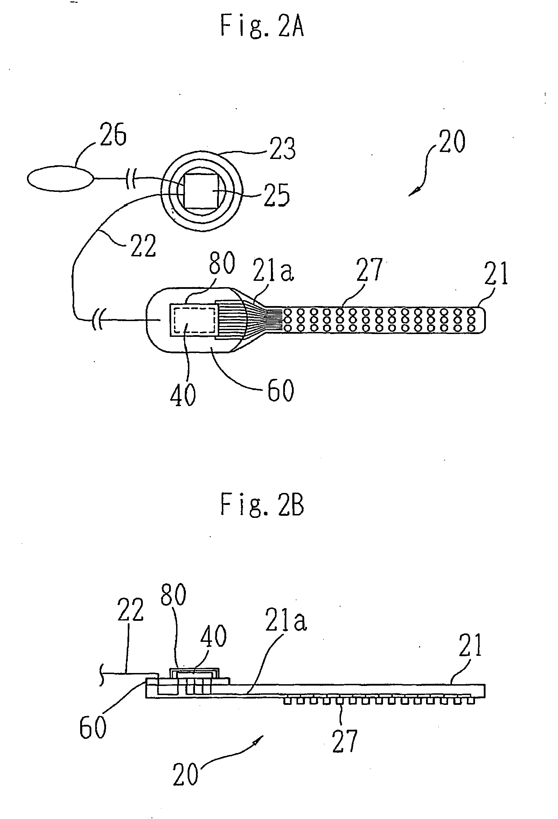

Embodiments of the present invention will be described with reference to the accompanying drawings. FIG. 1 is a schematic view of an external device 10 of a vision regeneration (revival) assisting apparatus 1 according to an embodiment of the present invention. FIG. 2A and FIG. 2B are schematic views of an internal device 20 of the apparatus 1. FIG. 2A is a plan view when the internal device 20 is seen from an electrode disposition side, and FIG. 2B is a sectional view when the electrode disposition side is arranged (oriented) downwardly and the internal device 20 is seen from a lateral side.

The external device 10 comprises: a visor 11 worn by a patient; a photographing unit 12 made of a camera such as a CCD camera mounted on the visor 11; an external unit 13; and a transmitter unit 14 made of a primary coil. The visor 11 is formed in an eyeglass shape, and is used after being attached in front of a patient's eye. In addition, the photographing unit 12 is mounted on a front face of the visor 11, and photographs an object to be visually recognized by the patient.

The external unit 13 comprises: a processor unit (signal converter unit) 13a having an arithmetical processing circuit such as a CPU; and a power unit (battery) 13b for supplying electrical power to the apparatus 1 (external device 10 and internal device 20) . The processor unit 13a image-processes image data obtained by the photographing unit 12, and then, converts the image data to electrical stimulation pulse signal data.

The transmitter unit 14 transmits to the internal device 20, the electrical stimulation pulse signal data converted by the processor unit 13a, and the electrical power data from the power unit 13b via the processor unit 13a as an electromagnetic wave. At the center of the transmitter unit 14, a magnet 15 is mounted which improves data transmission efficiency by the transmitter unit 14 and which is used for fixing a position relevant to a receiver unit 23 described below.

The internal device 20 comprises: a substrate 21 on which a plurality (a number) of electrodes 27 for applying electrical stimulation to cells that compose a retina are disposed (arranged); a cable 22; the receiver unit 23 made of a secondary coil; a control unit 25; an indifferent electrode 26; and a multiplexer 40.

The substrate 21 is formed in a shape of an elongated thin plate of materiel such as insulating, biocompatible and bendable resin with predetermined thickness such as polyimide or parylene. In addition, in the substrate 21, a lead wire 21a is formed of material such as conductive and biocompatible metal such as gold or platinum, and is provided therein by well-known photolithography. In addition, one face of the substrate 21 is defined as a face on which an electrode is disposed (formed), and the lead wire 21a is exposed at the disposed position. The electrode 27 is formed of material such as conductive and biocompatible metal such as gold or platinum at this exposed lead wire 21a by technique such as sputtering technique. In other words, each electrode 27 is connected to the multiplexer 40 via individual lead wire 21a.

Each electrode 27 is disposed at substantially equal intervals on one face of the substrate 21, and then, an electrode array is formed. The number of electrodes 27 is determined depending on resolution at the time of vision regeneration (revival), and several tens to several hundreds of electrodes are disposed. Many more electrodes may be disposed unless a problem occurs with an electrode disposition space, wiring technique of a lead wire or the like.

The receiver unit 23 receives the electrical stimulation pulse signal data and the electrical power data sent from the transmitter unit 14. At the center of the receiver unit 23 as well, a magnet 24 is mounted, which improves data receiving efficiency by the receiver unit 23 and which is used for fixing a position relevant to the transmitter unit 14.

The control unit 25 has circuits such as: a circuit for separating the electrical stimulation pulse signal data and the electrical power data received by the receiver unit 23; a circuit for obtaining an electrical stimulation pulse signal and a control signal of the multiplexer 40, based on the electrical stimulation pulse signal data; a circuit for transmitting the electrical stimulation pulse signal and the control signal to the multiplexer 40; anda circuit for obtaining driving power of an internal device 20 (such as the control unit 25 and the multiplexer 40), based on the electrical power data.

The multiplexer 40 is disposed on the other face of the substrate 21 (opposite face to the face on which the electrode 27 is disposed), and distributes the electrical stimulation pulse signal sent from the control unit 25 to an electrode 27 required for visual recognition of the object, based on the control signal sent from the control unit 25.

The cable 22 is coated with material such as insulating and biocompatible resin, and then, the control unit 25 and the multiplexer 40 are electrically connected to each other.

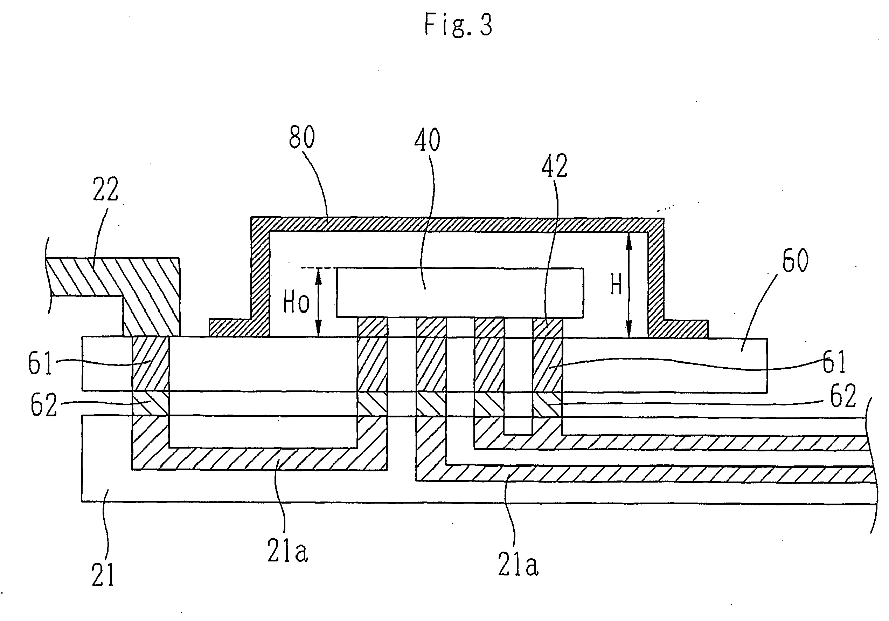

FIG. 3 is a schematic cross section of the vicinity of the multiplexer 40 of the internal device 20. The multiplexer 40 is disposed and united on a base 60 disposed and united on the substrate 21, and is covered with a cap 80. The multiplexer 40 is united to the base 60 while a face having a circuit pattern formed thereon is formed at the base 60 side. The base 60 is formed in a shape of a thin plate of material such as sealing, insulating and biocompatible ceramics such as alumina, glass, or ferrite. In addition, in the base 60, a lead wire 61 electrically connected to a terminal of the circuit pattern of the multiplexer 40 is formed so as to penetrate straightway or in a bent manner in a thickness direction of the base 60.

Fabrication of the base 60 will be described in this context (assuming that the base 60 is made of alumina). First, alumina is spread in a plate shape with thickness of 10µm, and a hole is drilled at a position corresponding to that of the terminal of the circuit pattern of the multiplexer 40 at the time of uniting to the base 60. Next, in this hole, paste obtained by melting material such as conductive and biocompatible metal such as gold or platinum serving as the lead wire 61 in a volatile solvent, is provided in a gapless manner. Next, an alumina plate is heated in a furnace at high temperature, whereby alumina is contracted and the paste solvent evaporates, the metal is hardened, and then, the lead wire 61 is formed in intimate contact with the alumina plate. At last, the alumina plate is reformed by cutting or grinding, and then, the base 60 is completed.

In the case where material such as non-biocompatible metal is used for the lead wire 61, material such as biocompatible resin is coated at a portion such as an exposed portion from the base 60 of the lead wire 61.

The cap 80 is formed in a hat shape of material such as sealing and biocompatible metal or ceramics such as gold, platinum, titanium, alumina, glass, or ferrite. It is preferable that thickness of the cap 80 is small in order to make compact the internal device 20, and thus, it is preferable that the thickness is in a range of several tens to 500µm, and it is more preferable that the thickness is in a range of 100 to 200µm. In addition, height H of an inside (internal space) of the cap 80 is slightly higher than a top face of the multiplexer 40 united to the base 60.

Next, hermetic sealing of the multiplexer 40 will be described below.

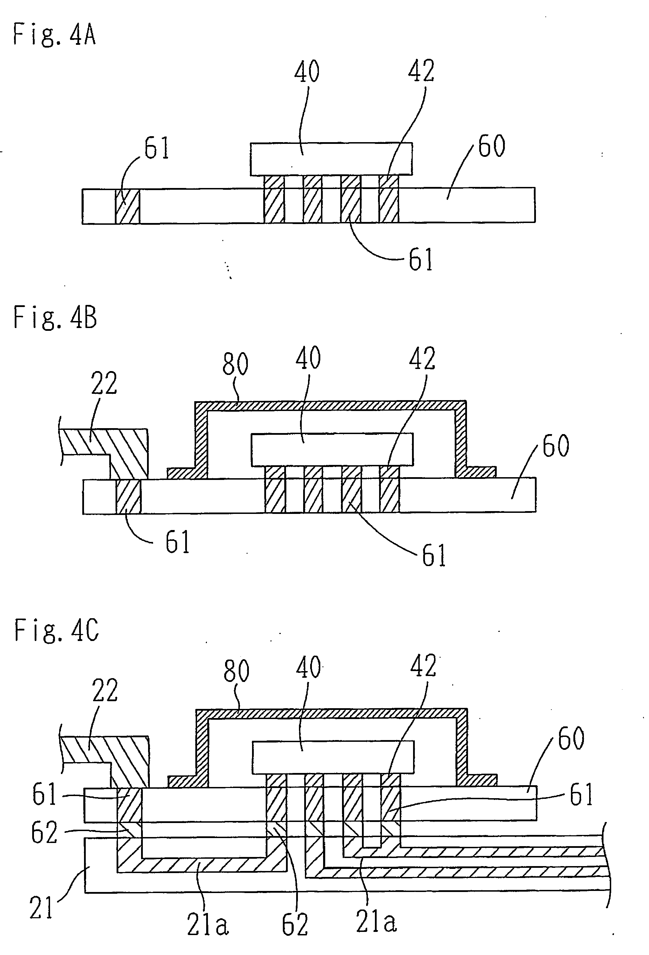

First, as shown in FIG. 4A, the multiplexer 40 is united to the base 60 by well-known flip chip attaching (flip chip bonding). In other words, a bump 42 formed at the multiplexer 40 is united to the lead wire 61 of the base 60. Insulating filler is filled in a gap between the multiplexer 40 and the base 60. It is preferable that this filler has a bonding (adhesive) property.

Next, as shown in FIG. 4B, the cap 80 is united to the base 60 by bonding, welding (including brazing and soldering) or the like under an atmosphere containing inert gas such as argon gas or nitrogen gas. In this manner, the inert gas is charged in the internal space of the cap 80. In addition, the cable 22 and the lead wire 61 of the base 60 are united with each other. In the case where either of the base 60 or the cap 80 is made of ceramics, well-known metalizing may be carried out at the uniting portion. In addition, material employed for uniting such as bonding or welding is also biocompatible material.

The multiplexer 40 is hermetically sealed by uniting the cap 80 to the base 60. Insulating filler instead of the inert gas may be filled in the internal space of the cap 80. In addition, uniting under a vacuum condition may be carried out.

At last, as shown in FIG. 4C, the base 60 is united to the substrate 21. In other words, the lead wire 61 of the base 60 and the lead wire 21a of the substrate 21 are united with each other. In FIG. 4C, an exposed portion from the substrate 21 of the lead wire 21a is united with a bump 62 formed at the lead wire 61, whereas an exposed portion from the base 60 of the lead wire 61 may be united with a bump formed at the lead wire 21. Insulating and biocompatible filler is filled in a gap between the base 60 and the substrate 21. It is preferable that this filler be has a bonding (adhesive) property.

Following the process described above, elements other than the electrodes 27 are coated with material such as insulating and biocompatible resin, whereby further sealing is carried out.

Inthisway, the base is formed of sealing and insulating material, and then, the lead wire is wired at the time of fabrication of the base, whereby the lead wire coming into contact with the base can be easily wired in comparison with a case in which the lead wire is wired after fabrication of the base. In addition, the multiplexer can be easily hermetically sealed.

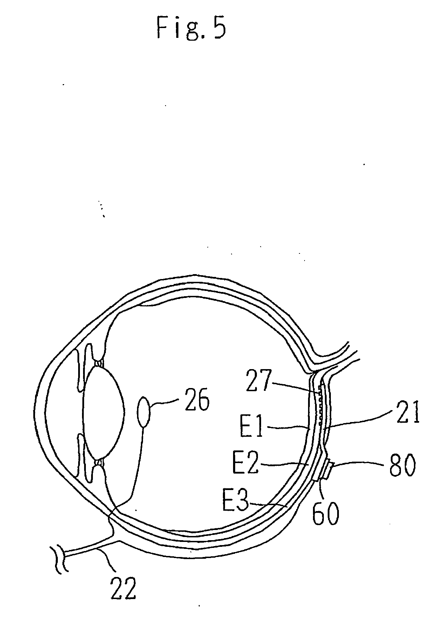

In the internal device 20 (substrate 21) of the embodiment, as shown in FIG. 5, a front side (a side at which the electrode 27 is disposed) is installed in an eye (for example, between a choroidea E2 and a sclera E3) and a rear side (a side at which the multiplexer 40 is disposed) is installed outside of the eye (outside of the sclera E3), whereas the multiplexer 40 is disposed on an opposite face of the substrate 21 to the face on which the electrode 27 is disposed. Thus, the multiplexer 40 is not in abutment against the sclera E3 at the time of installation of the internal device 20. Therefore, the internal device 20 can be easily installed.

The installation position of the internal device 20 is not limited to the position described above, and may be such that the electrode 27 can be installed at a position at which cells that compose a retina E1 can be preferably electrically stimulated.

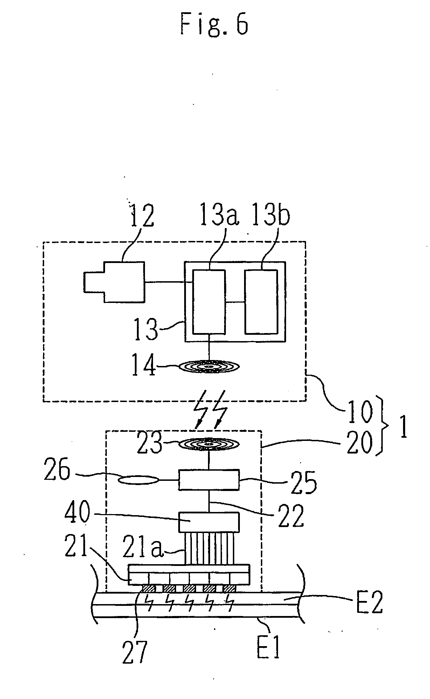

Next, an operation of the apparatus 1 having the configuration mentioned above will be described below. FIG. 6 is a schematic block diagram of a control system of the apparatus 1.

The image data obtained by the photographing unit 12 is inputted to the processor unit 13a; the inputted data is converted to a signal (electrical stimulation pulse signal data) of a predetermined bandwidth by the processor unit 13a; and then, the converted signal is transmitted to an internal device 20 by the transmitter unit 14.

In addition, the electrical power supplied from the power unit 13b is converted to a signal (electrical power data) of a bandwidth which is different from that of the electrical stimulation pulse signal data by the processor unit 13a, and then, the converted signal is transmitted to the internal device 20 together with the electrical stimulation pulse signal data by the transmitter unit 14.

The electrical stimulation pulse signal data and the electrical power data transmitted from the internal device 10 are received by the receiver unit 23, and then, the received data are inputted to the control unit 25. The inputted data are separated into the electrical stimulation pulse signal data and the electrical power data by the control unit 25, the electrical stimulation pulse signal and the control signal of the multiplexer 40 are generated based on the electrical stimulation pulse signal data, and then, the generated signals are sent to the multiplexer 40.

The multiplexer 40 distributes the electrical stimulation pulse signal transmitted from the control unit 25 to the electrode 27 required for visual recognition of the object, based on the.control signal sent from the control unit 25. The electrical stimulation pulse signal outputted from the electrode 27 stimulates the cells that compose the retina E1. In this manner, the patient can visually recognize the object photographed by the photographing unit 12.

In the case where the cap 80 is formed of material such as insulating ceramics, the height H inside of the capo 80 may be equal to height Ho of the multiplexer 40 and the bump 42. In other words, the cap 80 may come into contact with the multiplexer 40. On the other hand, in the case where the cap 80 is formed of material such as non-insulating metal, the height H inside of the cap 80 must be slightly higher than the height Ho of the multiplexer 40 and the bump 42. In other words, the cap 80 must be spaced from the multiplexer 40. However, in the case where the cap 80 is formed of the metal, thickness can be formed to be smaller in comparison with a case in which the cap 80 is formed of the ceramics.

The shapes of the base 60 and the cap 80 are not limited to the shape mentioned above and may be formed to such shape that the multiplexer 40 can be hermetically sealed with the base 60 and the cap 80. For example, the base 60 may be formed in a hat shape and the cap 80 may be formed in a plate shape.

A method for uniting the multiplexer 49 to the base 60 is not limited to flip chip attaching, and may be a well-known method such as wire bonding.

## Claims

1. A vision regeneration assisting apparatus for regenerating vision by applying electrical stimulation to cells that compose a retina, the apparatus comprising:
a substrate (21) formed in a thin plate shape of insulating and bendable material, provided with a plurality of first lead wires (21a) being wired therein and an electrode (27) for outputting an electrical stimulation pulse signal being provided at one end of each of the first lead wires;
a multiplexer (40) for distributing the electrical stimulation pulse signal to each electrode;
a base (60) formed of sealing and insulating material, one face of which being united to the substrate, and the other face of which being united with the multiplexer, and provided with a plurality of second lead wires (61) for connecting the multiplexer and each of the first lead wires in the substrate being wired therein; and
a cap (80) formed of sealing material, and united to the base so that the multiplexer is to be hermetically sealed.

2. The vision regeneration assisting apparatus according to claim 1, wherein:
the base is formed of sealing and insulating ceramics; and
the cap is formed of sealing metal or sealing ceramics.

3. The vision regeneration assisting apparatus according to claim 1 or 2, wherein, on the base, the multiplexer is united by flip chip attaching.

4. The vision regeneration assisting apparatus according to one of claims 1 to 3, wherein:
the substrate is formed in a shape of an elongated thin plate;
the electrode is provided at a front side of the substrate; and
the base is united to a rear side of the substrate.

5. The vision regeneration assisting apparatus according to claim 4, wherein:
the electrode is provided on one face of the substrate; and
the base is united to the other face of the substrate.

6. The vision regeneration assisting apparatus according to claim 5, wherein:
the front side of the substrate is installed by inserting into an eye; and
the rear side of the substrate is installed outside of the eye.

7. The vision regeneration assisting apparatus according to one of claims 1 to 6, wherein the apparatus is an extra-body imaging type apparatus, and further, has a photographing unit for photographing an obj ect to be visually recognized by a patient outside of a patient's body.
